Europäisches Patentamt

(19) European Patent Office

Office européen des brevets

(11) **EP 1 117 716 B1**

(12) **EUROPÄISCHE PATENTSCHRIFT**

(45) Veröffentlichungstag und Bekanntmachung des Hinweises auf die Patenterteilung:
**17.11.2004  Patentblatt 2004/47**

(51) Int Cl.⁷: **C08G 18/38**, C08G 18/78, C09D 201/06

(21) Anmeldenummer: **99944542.2**

(22) Anmeldetag: **27.08.1999**

(86) Internationale Anmeldenummer:
**PCT/EP1999/006327**

(87) Internationale Veröffentlichungsnummer:
**WO 2000/012578 (09.03.2000 Gazette 2000/10)**

(54) **OLIGOMERE UND HOCHMOLEKULARE UMSETZUNGSPRODUKTE VON ALLOPHANSÄUREESTERN MIT NUCLEOPHILEN VERBINDUNGEN SOWIE IHRE VERWENDUNG**

OLIGOMERIC AND HIGH-MOLECULAR CONVERSION PRODUCTS OF ALLOPHANIC ACID ESTERS WITH NUCLEOPHILIC COMPOUNDS , AND THE USE THEREOF

PRODUITS OLIGOMERES ET DE POIDS MOLECULAIRE ELEVE RESULTANT DE LA REACTION D'ESTERS D'ACIDE ALLOPHANIQUE AVEC DES COMPOSES NUCLEOPHILES ET LEUR UTILISATION

(84) Benannte Vertragsstaaten:
**DE ES FR IT**

(30) Priorität: **29.08.1998  DE 19839453**

(43) Veröffentlichungstag der Anmeldung:
**25.07.2001  Patentblatt 2001/30**

(73) Patentinhaber: **BASF Coatings AG**
**48165 Münster (DE)**

(72) Erfinder:
• **RINK, Heinz-Peter**
  **D-48153 Münster (DE)**
• **JUNG, Werner**
  **D-59387 Ascheberg (DE)**
• **RÖCKRATH, Ulrike**
  **D-48308 Senden (DE)**
• **SAVINO, Tom**
  **Northville, MI 48167 (US)**

(74) Vertreter: **Fitzner, Uwe, Dr. et al**
**Dres. Fitzner & Münch**
**Rechts- und Patentanwälte**
**Lintorfer Strasse 10**
**40878 Ratingen (DE)**

(56) Entgegenhaltungen:
**EP-A- 0 059 400          EP-A- 0 079 515**
**EP-A- 0 133 274          DE-A- 19 644 932**

• **KRICHELDORF ET AL: "Polymers of carbonic acid -7. Synthesis and characterization of aliphatic polyallophanates" EUROPEAN POLYMER JOURNAL, Bd. 27, Nr. 10, 1991, Seiten 1039-1044, XP000243871 Marsh Barton, UK**

**Beschreibung**

Die vorliegende Erfindung betrifft neue oligomere und hochmolekulare Umsetzungsprodukte von Allophansäureestern mit nucleophilen Verbindungen.

Desweiteren betrifft die vorliegende Erfindung die Verwendung dieser Umsetzungsprodukte und Verbindungen in Beschichtungsmitteln, Folien, Klebstoffen und Dichtungsmassen. Außerdem betrifft die vorliegende Erfindung neue Beschichtungsmittel, Folien, Klebstoffe und Dichtungsmassen, welche die neuen oligomeren und/oder hochmolekularen Umsetzungsprodukte von Allophansäureestern mit nucleophilen Verbindungen und/oder die neuen niedermolekularen, oligomeren und hochmolekularen Verbindungen mit Allophanatseiten- und/oder -endgruppen, enthalten. Nicht zuletzt betrifft die vorliegende Erfindung neue Verfahren zur Herstellung dieser Umsetzungsprodukte oder Verbindungen.

Klarlacke für den Automobilsektor, welche Oligomere und/oder Polymere mit Carbamatseiten- und/oder -endgruppen (-O-C(O)-NH$_2$) enthalten, sind aus den Patentschriften EP-A- 0 594 071, EP-A- 0 594 068, EP-A- 0 594 142, WO 94/10212, WO 94/10211, WO 94/10213, DE-C- 36 34 780 und US -A- 5 098 947 bekannt. Sie können nach unterschiedlichen, in diesen Patentschriften beschriebenen Verfahren hergestellt werden. Es hat sich indes bewährt, diese Carbamatseiten- und/oder -endgruppen enthaltenden Oligomere und Polymere durch Umsetzung aus hydroxylgruppenhaltigen Oligomeren und Polymeren mit Methylcarbamat herzustellen. Die Reaktivität von Methylcarbamat ist indes in manchen Fällen der Transcarbamatisierung vergleichsweise niedrig, so daß höhere Temperaturen angewandt werden müssen, um höhere Reaktiongeschwindigkeiten zu erzielen. Die höheren Temperaturen können jedoch dazu führen, daß die Produkte thermisch geschädigt werden, wonach sie für eine Verwendung z. B. in Klarlacken nicht mehr in Betracht kommen.

Niedermolekulare und oligomere Verbindungen, welche Carbamatseiten- und/oder - endgruppen (-HN-C(O) -O-R) enthalten, werden in den Patentschriften US-A-4 710 542 und EP -B- 0 245 700 sowie in dem Artikel von B. Singh und Mitarbeiter "Carbamylmethylated Melamines, Novel Crosslinkers for the Coatings Industry" in Advanced Organic Coatings Science and Technology Series, 1991, Band 13, Seiten 193 bis 207 beschrieben. Bei diesen Verbindungen handelt es sich um Umsetzungsprodukte von Carbamaten, insbesondere Alkylcarbamaten, und Melaminharzen. Diese Carbamatseiten- und/oder -endgruppen (-HN-C(O)-O-R) sind nicht mehr in der Lage, mit den Carbamatseiten- und/oder -endgruppen (-O-C(O)-NH$_2$) Vemetzungsreaktionen einzugehen. Diese niedermolekularen und oligomeren Verbindungen werden deshalb auch als "partiell defunktionalisierte Aminoplastharze" bezeichnet.

Niedermolekulare und oligomere Verbindungen, welche Carbamatseiten- und/oder - endgruppen (-HN-C(O) -O-R-O-C(O)-NH$_2$) enthalten, werden in den Patentschriften US-A-5 336 566 und EP-A-0 622 387 beschrieben. Diese Verbindungen werden aus Polyisocyanaten und Hydroxyalkylcarbamaten hergestellt. Sie gehen vor allen Vernetzungsreaktionen mit Aminoplastharzen ein.

Niedermolekulare Allophanatester sind bekannt aus den Patentschriften EP-A-0 133 274, EP-a-0 079 515 und DE-A-196 44 932. Verbindungen mit Allophanat-Endgruppen sind aus Kricheldorf et al. "Polymers of carbonic acid -7. Synthesis and characterization of aliphatic polyallophanates", Europ. Polymer J., Bd. 27, Nr. 10, 1991, S. 1039-44 bekannt.

Aufgabe der vorliegenden Erfindung ist es, neue oligomere und hochmolekulare Umsetzungsprodukte zu finden, welche eine Alternative zu den bisher bekannten Carbamatseiten- und/oder -endgruppen enthaltenden niedermolekularen, oligomeren und hochmolekularen Umsetzungsprodukten und/oder Verbindungen bieten und eine höhere Reaktivität als diese und zumindest dieselben vorteilhaften Eigenschaften wie diese aufweisen. Die neuen Umsetzungsprodukte sollen als neue vorteilhafte Vernetzer, und die neuen oligomeren und hochmolekularen Umsetzungsprodukte sollen als neue vorteilhafte Bindemittel für Beschichtungsmittel, Klebstoffe und Dichtungsmassen oder für die Herstellung von Folien verwendbar sein.

Außerdem ist es die Aufgabe der vorliegenden Erfindung neue Beschichtungsmittel, Klebstoffe, Dichtungsmassen und Vorstufen von Folien bereitzustellen, welche eine neue Vernetzungschemie aufweisen und eine vorteilhafte Alternative zu den bisher bekannten Systemen bieten.

Desweiteren ist es Aufgabe der vorliegenden Erfindung, neue einfache und elegante Verfahren zur Herstellung der neuen oligomeren und hochmolekularen Umsetzungsprodukte zu finden.

Demgemäß wurden die neuen oligomeren und hochmolekularen Umsetzungsprodukte von Allophansäureestern mit nucleophilen Verbindungen gefunden.

Außerdem wurden die neuen Beschichtungsmittel, Folien, Klebstoffe und Dichtungsmassen, welche die neuen niedermolekularen, oligomeren und/oder hochmolekularen Umsetzungsprodukte von Allophansäureestern mit nucleophilen Verbindungen enthalten, gefunden.

Darüberhinaus wurden

A) ein neues Verfahren zur Herstellung der neuen oligomeren und hochmolekularen Umsetzungsprodukte von Allophansäureestern mit nucleophilen Verbindungen und/oder von oligomeren und hochmolekularen Verbindungen mit Allophanatseiten- und/oder - endgruppen durch Transallophanatisierung von hydroxylgruppenhaltigen Oligomeren und/oder Polymeren (Nucleophilen) mit Allophansäurealkyl- und arylestem,

B) ein neues Verfahren zur Herstellung von niedermolekularen und oligomeren Verbindungen durch Umsetzung von Aminoplastharzen mit Allophansäurealkyl- und -arylestern sowie

C) ein weiteres neues Verfahren zur Herstellung von niedermolekularen und oligomeren Verbindungen durch Umsetzung von Polyisocyanaten mit Hydroxyalkylallophanaten gefunden.

Im Hinblick auf den Stand der Technik war es überraschend und nicht vorherzusehen, daß die der Erfindung zugrundeliegende Aufgabe mit Hilfe von Allophanaten der allgemeinen Formel I und Allophanatgruppen der allgemeinen Formeln II, III und IV gelöst werden konnte:

$$\text{R-O-C(O)-NH-C(O)-NH2} \qquad \text{(I)}$$

$$\text{-O-C(O)-NH-C(O)-NH}_2 \qquad \text{(II)}$$

$$\text{-CH}_2\text{-NH-C(O)-NH-C(O)-OR}^1 \qquad \text{(III)}$$

$$\text{- O-R}^2\text{-O-C(O)-NH-C(O)-NH}_2 \qquad \text{(IV)}$$

In der allgemeinen Formel III bezeichnet der Rest $R^1$

-   eine Alkyl- oder Cycloalkylgruppe, insbesondere eine $C_1$- bis $C_{10}$-Alkylgruppe oder eine $C_5$- bis $C_{10}$-Cycloalkylgruppe; Beispiele geeigneter erfindungsgemäß zu verwendender Gruppen dieser Art sind Methyl-, Ethyl-, Propyl-, Isopropyl-, n-Butyl-, Isobutyl-, tert.-Butyl-, n-Pentyl-, Neopentyl-, n-Hexyl-, 2-Methyl-hexyl-, n-Heptyl-, 2-Ethyl-hexyl-, n-Octyl-, Isooctyl-, n-Nonyl-, n-Decyl-, Cyclopentyl-, Cyclohexyl-, Cycloheptyl-, Indenyl- oder Decalinylreste;

-   eine Arylgruppe, insbesondere eine Phenylgruppe;

-   einen der vorstehend genannten Reste mit im wesentlichen inerten Gruppen, welche nicht mit Vernetzungsmitteln oder Bindemitteln reagieren; Beispiele geeigneter Gruppen dieser Art sind Halogenatome wie Chlor- oder Fluoratome, aromatische Reste, Nitrogruppen oder Alkyl- oder Arylethergruppen; sowie

-   eine der vorstehend genannten Gruppen, welche mindestens eine freie Hydroxylgruppe aufweisen, insbesondere solche, welche sich von niedermolekularen linearen oder verzweigten oder von cycloaliphatischen Polyolen ableiten; Beispiele geeigneter Polyole sind Neoalkohole, Butylethylpropandiol-1,3, 2-Methylpropandiol-1,3, Hexandiol-1,6, Octandiol-1,8, 2,4-Diethyloctandiol-1,3, 2-Ethylhexandiol-1,3, 1,4-Dimethylolcyclohexan, Trimethylolpropan, Glycerin, Diglycerin, Polyglycerin, Pentaerythrit, Dipentaerythrit oder Homopentaerythrit.

In der allgemeinen Formel IV bezeichnet der Rest $R^2$

-   lineare oder verzweigte Alkandiylreste oder Cycloalkandiylreste, insbesondere Methylen-, Ethylen-, Propylen-1,2-, Tetramethylen-, Pentamethylen-, Hexamethylen-, Heptamethylen-, Cyclohexan-1,3-, -1,2- oder -1,4- diyl-, Butylethylpropan-1,3-diyl -, 2-Methylpropan-1,3-diyl-, 2,4-Diethyloctan-1,3- diyl-, 2-Ethylhexan-1,3-diyl- oder Cyclohexan-1,4-dimethylen-Reste;

-   Arylenreste, insbesondere 1,2-, 1,4- und 1,4-Phenylenreste;

-   die vorstehend genannten Diyl- und Ylenreste, welche die oben genannten inerten Gruppen tragen, die nicht mit Vernetzungsmitteln oder Bindemitteln reagieren; sowie

-   Diylreste, welche noch mindestens eine freie Hydroxylgruppe aufweisen und sich von Triolen und Polyolen, insbesondere von Trimethylolpropan, Glycerin, Diglycerin, Polyglycerin, Pentaerythrit, Dipentaerythrit oder Homopentaerythrit, ableiten.

Mit Hilfe dieser Allophanate und Allophanatgruppen wird eine neuartige Vernetzungschemie bereitgestellt, welche Ergebnisse liefert, die denen der Vernetzungschemie auf der Basis von Carbamaten und Aminoplastharzen ebenbürtig, wenn nicht gar überlegen ist.

Im folgenden werden der Kürze halber die Allophanate der allgemeinen Formel I als "Allophanate I" und die Allophanatgruppen der allgemeinen Formeln II, III und IV je nach dem als "Allophanatgruppen II, III und IV" bezeichnet.

Außerdem werden im folgenden die neuen oligomeren und hochmolekularen Umsetzungsprodukte von Allophansäurestern mit Nucleophilen der Kürze halber je nach dem als "oligomere erfindungsgemäße" oder "hochmolekulare erfindungsgemäße Umsetzungsprodukte", und die niedermolekularen, oligomeren und hochmolekularen Verbindungen, welche Allophanatseitenund/oder -endgruppen enthalten, werden entsprechend der Kürze halber je nach dem als "niedermolekulare", oligomere" oder "hochmolekulare Verbindungen" bezeichnet.

Desweiteren werden im folgenden der Kürze halber das neue Verfahren zur Herstellung der oligomeren und hochmolekularen erfindungsgemäßen Umsetzungsprodukte und Verbindungen durch Transallophanatisierung als "erfindungsgemäßes Verfahren A", das neue Verfahren zur Herstellung der niedermolekularen und oligomeren . Verbindungen durch Umsetzung von Aminoplastharzen mit Allophansäurealkyl- und arylestem als "erfindungsgemäßes Verfahren B" und das neue Verfahren zur Herstellung der niedermolekularen und oligomeren Verbindungen durch Umsetzung von Polyisocyanaten mit Hydroxyalkyl- und -arylallophanaten als "erfindungsgemäßes Verfahren C" bezeichnet.

Im Rahmen der vorliegenden Erfindung werden die Begriffe "niedermolekular", "oligomer" und "hochmolekular" in ihrer geläufigen Bedeutung verwendet. D. h., der Begriff "oligomer" bedeutet, daß die betreffende Verbindung im Mittel aus 3 bis in etwa 10 sich wiederholenden identischen Grundbausteinen aufgebaut ist und somit ein Oligomer darstellt. Demgemäß bedeutet der Begriff "hochmolekular" , daß die betreffende Verbindung im Mittel aus mehr als 10 sich wiederholenden identischen Grundbausteinen aufgebaut ist und somit ein Polymer darstellt, das gegebenenfalls auch in Form hochvernetzter Teilchen vorliegen kann.

Die oligomeren und hochmolekularen erfindungsgemäßen Umsetzungsprodukte werden durch die Umsetzung von Nucleophilen mit Allophansäurealkyl- und arylestern gebildet. Je nach der Natur des Nucleophils können die unterschiedlichsten funktionellen Gruppen resultieren. Hierbei kann es sich indes auch um die Allophanatgruppen II, III und IV, insbesondere aber um die Allophanatgruppen II, handeln.

Bei den oligomeren und hochmolekularen erfindungsgemäßen Verbindungen handelt es sich um Oligomere und Polymere, welche die Allophanatgruppen II, III und IV als Seiten- und/oder Endgruppen enthalten. Erfindungsgemäß ist es indes von Vorteil, wenn die oligomeren und hochmolekularen erfindungsgemäßen Verbindung lediglich mindestens zwei, vorzugsweise mindestens drei der Allophanatgruppen II enthalten.

Wenn die oligomeren und hochmolekularen erfindungsgemäßen Umsetzungsprodukte die Allophanatgruppen II, III und IV, insbesondere aber die Allophanatgruppen II, als Seiten- und/oder als Endgruppen enthalten, handelt es sich folgerichtig um die oligomeren und hochmolekularen erfindungsgemäßen Verbindungen. Da sich in diesen Fällen die Herstellung der oligomeren und hochmolekularen erfindungsgemäßen Umsetzungsprodukte nicht von der Herstellung der oligomeren und hochmolekularen Verbindungen unterscheidet, werden beide im folgenden zusammen unter den Verbindungen abgehandelt.

In speziellen Fällen ist es erfindungsgemäß von Vorteil, wenn die niedermolekularen und oligomeren Verbindungen lediglich mindestens zwei, insbesondere mindestens drei, der Allophanatgruppen III oder IV enhalten.

Zwar kann die Herstellung der oligomeren und hochmolekularen erfindungsgemäßen Verbindungen mit Allophanatgruppen II nach beliebigen Methoden erfolgen, indes ist es erfindungsgemäß von Vorteil, sie nach dem erfindungsgemäßen Verfahren A herzustellen. Hierzu werden Oligomere und Polymere, welche mindestens zwei, vorzugsweise mindestens drei primäre und/oder sekundäre, insbesondere aber primäre, Hydroxylgruppen aufweisen und damit auch Nucleophile im Sinne der vorliegenden Erfindung sind, mit Allophansäurealkyl- und arylestern I bei 30 bis 200 °C, vorzugsweise 50 bis 160 °C, besonders bevorzugt 60 bis 150 °C und insbesondere 80 bis 140 °C transallophanatisiert. Die Reaktion wird in Lösung oder in Masse, vorzugsweise in Lösung durchgeführt. Es empfiehlt sich, dem Reaktionsgemisch übliche und bekannte Inhibitoren wie Trialkylphosphite, insbesondere Trüsodecylphosphit, zuzusetzen. Außerdem ist es von Vorteil, übliche und bekannte Umesterungskatalysatoren wie Zinnverbindungen, insbesondere Dibutylzinndioxid, zu verwenden.

In der allgemeinen Formel I bezeichnet der Rest R $C_1$- bis $C_{10}$-Alkylreste, $C_5$- bis $C_{10}$-Cycloalkyl- oder Phenylreste. Beispiele geeigneter Reste dieser Art werden vorstehend bei der Beschreibung des Restes $R^1$ der Formel III aufgeführt. Erfindungsgemäß sind die $C_1$- bis $C_5$-Alkylreste bei der Transallophanatisierung von Vorteil und werden deshalb bevorzugt verwendet. Beispiele geeigneter, erfindungsgemäß zu verwendender Allophanate I sind demnach Allophansäuremethyl-, Allophansäureethyl-, Allophansäurepropyl-, Allophansäurebutyl-, Allophansäurepentylester oder Allophansäurephenylester, von denen Allophansäuremethyl- und Allophansäureethylester besonders vorteilhaft sind und deshalb erfindungsgemäß besonders bevorzugt verwendet werden.

Als erfindungsgemäß zu verwendende Oligomere und Polymere, welche mindestens zwei, vorzugsweise mindestens drei primäre und/oder sekundäre, insbesondere aber primäre, Hydroxylgruppen aufweisen (Nucleophile), kommen vorzugsweise lineare und/oder verzweigte und/oder blockartig, kammartig und/oder statistisch aufgebaute Poly(meth)acrylate, Polyester, Polyurethane, acrylierte Polyurethane, acrylierte Polyester, Polylactone, Polycarbonate, Polyether, (Meth)Acrylatdiole, Polyharnstoffe oder oligomere Polyole in Betracht.

Neben den Hydroxylgruppen können die Oligomere und Polymere noch andere funktionelle Gruppen wie Acryloyl-, Ether-, Amid -, Imid -, Thio -, Carbonat- oder Epoxidgruppen enthalten.

Diese Oligomere und Polymere sind dem Fachmann bekannt, und zahlreiche geeignete Verbindungen sind am Markt erhältlich.

Erfindungsgemäß sind die oligomeren Polyole, die Polyacrylate, die Polyester und/oder die acrylierten Polyurethane von Vorteil und werden deshalb bevorzugt verwendet.

Beispiele für bevorzugte, erfindungsgemäß zu verwendende Oligomere und Polymere, welche mindestens zwei, vorzugsweise mindestens drei primäre und/oder sekundäre, insbesondere aber primäre, Hydroxylgruppen aufweisen, sind

1. Oligomere Polyole, welche aus Oligomeren, die durch Metathesereaktionen von acyclischen Monoolefinen und cyclischen Monoolefinen gewonnen werden, durch Hydroformylierung und anschließender Hydrierung erhältlich sind; Beispiele geeigneter cyclischer Monoolefine sind Cyclobuten, Cyclopenten, Cyclohexen, Cycloocten, Cyclohepten, Norbonen oder 7-Oxanorbonen; Beispiele geeigneter acyclischer Monoolefine sind in Kohlenwasserstoffgemischen enthalten, die in der Erdölverarbeitung durch Cracken erhalten werden ($C_5$-Schnitt); Beispiele geeigneter, erfindungsgemäß zu verwendender oligomerer Polyole weisen eine Hydroxylzahl (OHZ) von 200 bis 450, ein zahlenmittleres Molekulargewicht Mn von 400 bis 1000 und ein massenmittleres Molekulargewicht $M_w$ von 600 bis 1100 auf;

2. Polyacrylate mit einer Hydroxylzahl von 40 bis 240, vorzugsweise 60 bis 210, insbesondere 100 bis 200, einer Säurezahl von 0 bis 35, Glasübergangstemperaturen von -35 bis + 80 °C und zahlenmittlere Molekulargewichte $M_n$ von 1500 bis 300.000.

Die Glasübergangstemperaturen der Polyacrylate wird bekanntermaßen durch Art und Menge der eingesetzten Monomere bestimmt. Die Auswahl der Monomeren kann von Fachmann unter Zuhilfenahme der folgenden Formel V getroffen werden, nach welcher die Glasübergangstemperaturen näherungsweise berechnet werden können.

$$\frac{1}{T_g} = \sum_{n=1}^{n=x} \frac{W_n}{T_{g^n}} \quad ; \quad W_n = 1 \qquad (V)$$

Tg = Glasübergangstemperatur des Polyacrylatharzes
$W_n$ = Gewichtsanteil des n-ten Monomers
$T_{g_n}$ = Glasübergangstemperatur des Homopolymers aus dem n-ten Monomer
x = Anzahl der verschiedenen Monomeren

Maßnahmen zur Steuerung des Molekulargewichts (z. B. Auswahl entsprechender Polymerisationinitiatoren, Verwendung von Kettenübertragungsmitteln oder spezieller Verfahren der Polymerisation usw.) gehören zum Fachwissen und müssen hier nicht näher erläutert werden.

2.1 Besonders bevorzugte Polyacrylate sind herstellbar indem (a1) 10 bis 92, vorzugsweise 20 bis 60 Gew.-%, eines Alkyl- oder Cycloalkylmethacrylats mit 1 bis 18, vorzugsweise 4 bis 13 Kohlenstoffatomen im Alkyl- oder Cycloalkylrest oder Mischungen aus solchen Monomeren, (a2) 8 bis 60, vorzugsweise 12,5 bis 50,0 Gew.-%, eines Hydroxyalkylacrylats oder eines Hydroxyalkylmethacrylats mit 2 bis 4 Kohlenstoffatomen im Hydroxyalkylrest oder Mischungen aus solchen Monomeren, (a3) 0 bis 5, vorzugsweise 0,7 bis 3 Gew.-% Acrylsäure oder Methacrylsäure oder Mischungen aus diesen Monomeren und (a4) 0 bis 50, vorzugsweise bis zu 30 Gew.-%, von von (a1), (a2) und (a3) verschiedenen, mit (a1), (a2) und (a3) copolymerisierbaren ethylenisch ungesättigten Monomeren oder Mischungen aus solchen Monomeren zu Polyacrylaten der vorstehend angegebenen Spezifikation polymerisiert werden.

Beispiele geeigneter (a1)-Komponenten sind Methyl-, Ethyl-, Propyl-, n-Butyl-, Isobutyl-, tert.-Butyl-, Pentyl-, Hexyl-, Heptyl- oder 2-Ethylhexylacrylat oder -methacrylat sowie Cyclohexyl-, tert.-Butylcyclohexyl- oder Isobornylacrylat oder -methacrylat.

Beispiele geeigneter (a2)-Komponenten sind Hydroxyethyl-, Hydroxypropyl- oder Hydroxybutyl- oder Hydroxymethylcyclohexylacrylat oder -methacrylat oder Addukte von (Meth)Acrylsäure und Epoxiden wie Versaticsäure[R]-glycidylester.

Beispiele geeigneter (a4)-Komponenten sind Vinylaromaten wie Styrol, Vinyltoluol, alpha- Methylstyrol, alpha-Ethylstyrol, kernsubstituierte Diethylstyrole, Isopropylstyrol, Butylstyrol und Methoxystyrole; Vinylether wie Ethylvinylether, n-Propylvinylether, Isopropylvinylether, n-Butylvinylether oder Isobutylvinylether; Vinylester wie Vinylacetat, Vinylpropionat, Vinylbutyrat, Vinylpivalat oder der Vinylester der 2-Methyl-2-ethylheptansäure; oder Allylether wie Trimethylolpropanmono-, -di- oder -triallylether oder ethoxilierter oder propoxilierter Allylalkohol.

2.2 Weitere Beispiele besonders bevorzugter Polyacrylate werden in der europäischen Patentanmeldung EP-A-0 767 185 und den amerikanischen Patentschriften US-A- 5 480 493, 5 475 073 oder 5 534 598 beschrieben.

2.3 Weitere Beispiele besonders bevorzugter Polyacrylate werden unter der Marke Joncryl[R] vertrieben, wie etwa Joncryl[R] SCX 912 und 922,5.

2.4 Weitere Beispiele für besonders bevorzugte Polyacrylate sind solche, welche erhältlich sind, indem (a1) 10 bis 51 Gew.-%, vorzugsweise 25 bis 41 Gew.-%, 4-Hydroxy-n-butylacrylat oder -methacrylat oder eine Mischung hiervon, insbesondere aber 4-Hydroxy-n-butylacrylat, (a2) 0 bis 36 Gew.-%, vorzugsweise 0,1 bis 20 Gew.-%, eines von (a1) verschiedenen hydroxylgruppenhaltigen Esters der Acrylsäure oder der Methacrylsäure oder eines Gemisches hiervon, (a3) 28 bis 85 Gew.-%, vorzugsweise 40 bis 70 Gew.-%, eines von (a1) und (a2) verschiedenen, aliphatischen oder cycloaliphatischen Esters der Methacrylsäure mit mindestens vier Kohlenstoffatomen im Alkoholrest oder eines Gemisches solcher Monomeren, (a4) 0 bis 3 Gew.-%, vorzugsweise 0,1 bis 2 Gew.-%, einer ethylenisch ungesättigten Carbonsäure oder einer Mischung aus solchen Säuren und (a5) 0 bis 20 Gew.-%, vorzugsweise 5 bis 15 Gew.-%, eines von (a1), (a3) und (a4) verschiedenen ungesättigten Monomeren oder einer Mischung aus solchen Monomeren zu einem Polyacrylat mit einer Hydroxylzahl von 60 bis 200, vorzugsweise von 100 bis 160, einer Säurezahl von 0 bis 35 und einem zahlenmittleren Molekulargewicht $M_n$ von 1500 bis 10000 polymerisiert werden, wobei die Zusammensetzung der Komponente (a3) so gewählt wird, daß bei alleiniger Polymerisation dieser Komponente (a3) ein Polymethacrylat einer Glasübergangstemperatur von + 10 bis + 100 °C, vorzugsweise von + 20 bis + 60 °C, erhalten wird.

Beispiele geeigneter Komponenten (a2) sind Hydroxialkylester der Acrylsäure und Methacrylsäure wie Hydroxyethyl- oder Hydroxypropylacrylat oder -methacrylat, wobei die Wahl so zu treffen ist, daß bei alleiniger Polymerisation dieser Komponente (a2) ein Polyacrylat einer Glasübergangstemperatur von 0 bis + 80 °C, vorzugsweise von + 20 bis + 60 °C erhalten wird.

Beispiele geeigneter Komponenten (a3) sind aliphatische Ester der Methacrylsäure mit vier bis 20 Kohlenstoffatomen in Alkoholrest wie n-Butyl -, Isobutyl -, tert. - Butyl -, 2-Ethylhexyl-, Stearyl- und Laurylmethacrylat; oder cycloaliphatische Ester der Methacrylsäure wegen Cyclohexylmethacrylat.

Beispiele geeigneter Komponenten (a4) sind Acrylsäure und/oder Methacrylsäure.

Beispiele geeigneter Komponenten (a5) sind vinylaromatische Kohlenwasserstoffe wie Styrol, alpha-Alkylstyrol oder Vinyltoluol; Amide der Acrylsäure und Methacrylsäure wie Methacrylamid und Acrylamid; Nitrile der Acrylsäure und Methacrylsäure; Vinylether oder Vinylester, wobei die Zusammensetzung dieser Komponente (a5) vorzugsweise so zutreffend ist, daß bei alleiniger Polymerisation der Komponenten (a5) ein Polyacrylat mit einer Glasübergangstemperatur von + 70 bis + 120 °C, insbesondere von + 80 bis + 100 °C resultiert.

2.5 Die Herstellung dieser Polyacrylate ist allgemein bekannt und wird beispielsweise in dem Standardwerk Houben-Weyl, Methoden der organischen Chemie, 4. Auflage, Band 14/1, Seiten 24 bis 255, 1961, beschrieben.

3. Polyesterharze, welche herstellbar sind, indem (a1) mindestens eine cycloaliphatische oder aliphatische Polycarbonsäure, (a2) mindestens ein aliphatisches oder cycloaliphatisches Polyol mit mehr als zwei Hydroxylgruppen im Molekül, (a3) mindestens ein aliphatisches oder cycloaliphatisches Diol und (a4) mindestens eine aliphatische, lineare oder verzweigte gesättigte Monocarbonsäure in einem molaren Verhältnis von (a1) : (a2) : (a3) : (a4) = 1,0 : 0,2 bis 1,3 : 0,0 bis 1,1 : 0,0 bis 1,4, vorzugsweise 1,0 : 0,5 bis 1,2 : 0,0 bis 0,6 : 0, 2 bis 0,9 zu einem Polyester oder Alkydharz umgesetzt werden.

Beispiele geeigneter Komponenten (a1) sind Hexahydrophthalsäure, 1,4-Cyclohexandicarbonsäure, Endomethylentetrahydrophthalsäure, Oxalsäure, Malonsäure, Bernsteinsäure, Glutarsäure, Adipinsäure, Pimelinsäure, Korksäure, Azelainsäure oder Sebacinsäure.

Beispiele geeigneter Komponenten (a2) sind Pentaerythrit, Trimethylolpropan, Triethylolethan und Glycerin.

Beispiele geeigneter Komponenten (a3) sind Ethylenglykol, Diethylenglykol, Propylenglykol, Neopentylglykol, 2-Methyl-2-propylpropandiol-1,3, 2-Methyl-2-butylpropandiol-1,3, 2,2,4-Trimethylpentandiol-1,5, 2,2,5-Trimethylhexandiol-1,6, Hydroxypivalinsäureneopentylglykolester oder Dimethylolcyclohexan.

Beispiele geeigneter Komponenten (a4) sind 2-Ethylenhexansäure, Laurinsäure, Isooctansäure, Isononansäure oder Monocarbonsäuremischungen, welche aus Kokosfett oder Palmkernfett gewonnen werden.

Die Herstellung der erfindungsgemäß bevorzugt verwendeten Polyester und Alkydharze ist allgemein bekannt und wird beispielsweise in dem Standardwerk Ullmanns Encyklopädie der technische Chemie, 3. Auflage, Band 14, Urban & Schwarzenberg, München, Berlin, 1963, Seiten 80 bis 89 und Seiten 99 bis 105, sowie in den Büchern: "Résines Alkydes-Polyesters" von J. Bourry, Paris, Verlag Dunod, 1952, "Alkyd Resins" von C. R. Martens, Reinhold Publishing Corporation, New York, 1961, sowie "Alkyd Resin Technology" von T. C. Patton, Intersience Publishers, 1962, bschrieben.

4. Polyurethane, wie sie in den Patentschriften EP-A-0 708 788, DE-A-44 01 544 oder DE-A-195 34 361 beschrieben werden.

Die resultierenden oligomeren und hochmolekularen erfindungsgemäßen Umsetzungsprodukte und die oligomeren und hochmolekularen Verbindungen, welche die Allophanatgruppen II enthalten, eignen sich hervorragend als Bindemittel für Beschichtungsmittel, Klebstoffe und Dichtungsmassen.

Niedermolekulare und oligomere Verbindungen, welche Allophanatgruppen III enthalten, sind insbesondere Aminoplastharze auf der Basis von Melamin und/oder Benzoguanamin. Beispiele geeigneter Aminoplastharze sind Hexamethylolmelamin, Hexamethoximethylmelamin, Tetramethylolbenzoguanamin, Tetramethoximethylbenzoguanamin oder ihre oligomeren Kondensationsprodukte. Hierbei können alle Methylol- und/oder Methoximethylgruppen eines Ausgangsprodukts oder nur ein Teil hiervon zu Allophanatgruppen III umgewandelt sein.

Diese niedermolekularen und oligomeren Verbindungen können nach dem erfindungsgemäßen Verfahren B synthetisiert werden. Zu diesem Zweck werden die Aminoplastharze in Lösung oder in Masse mit Allophansäurealkylestern umgesetzt, welche außer den Resten R der allgemeinen Formel I auch noch die Reste $R^1$ der allgemeinen Formel III enthalten können. Erfindungsgemäß ist es von Vorteil, die Reaktion bei 50 bis 150 °C, vorzugsweise 60 bis 130 °C und insbesondere 80 bis 120 °C durchzuführen. Ein besonders guter Reaktionsverlauf wird gewährleistet, wenn man das Wasser und/oder das Methanol laufend aus dem Reaktionsgemisch entfernt, beispielsweise durch Destillation im Vakuum. Um die Reaktion zu beschleunigen, kann dem Reaktionsgemisch noch ein üblich und bekannter saurer Katalysator wie p-Toluolsulfonsäure zugesetzt werden.

Niedermolekulare und oligomere Verbindungen, welche Allophanatgruppen IV enthalten, sind insbesondere Umsetzungsprodukte von Polyisocyanaten mit Hydroxyalkyl- und -arylallophanaten der allgemeinen Formel VI:

$$\text{HO-R}^2\text{-O-C(O)-NH-C(O)-NH-C(O)-NH}_2 \qquad \text{(VI)}$$

Geeignete erfindungsgemäß zu verwendende Hydroxyalkylallophanate VI enthalten die vorstehend beschrie-benen Reste $R^2$, von denen der Ethylen- und der Trimethylenrest sowie der 1,4-Phenylenrest besonders vorteilhaft sind und deshalb ganz besonders bevorzugt verwendet werden.

Beispiele geeigneter erfindungsgemäß zu verwendender Polyisocyanate sind die üblichen und bekannten Polyisocyanate wie sie beispielsweise für die Herstellung von Zweikomponentenlacken verwendet werden, insbesondere solche, deren Isocyanatgruppen an aliphatische oder cycloaliphatische Reste gebunden sind. Beispiele vorteilhafter Polyisocyanate dieser Art sind Hexamethylendiisocyanat, Isophorondiisocyanat, Trimethylhexamethylendiisocyanat, Dicyclohexylmethandiisocyanate, 1,4- und 1,3-Bis(isocyanatomethyl)cycloalkane, insbesondere 1,4- u. 1,3 -Bis(isocyanatomethyl)cyclohexan oder die Addukte dieser Polyisocyanate an Polyole, insbesondere niedermolekulare Polyole wie Trimethylolpropan, oder die von diesen Polyisocyanaten abgeleitete isocyanuratgruppen-, uretdion-, allophanat- und/oder biuretgruppenhaltige Polyisocyanate.

Sind nur ein Teil der Methylol- und/oder Methoximethylgruppen eines Aminoplastharzes zu Allophanatgruppen III umgewandelt, können die betreffenden niedermolekularen und/oder oligomeren Verbindungen, welche die Allophanatgruppen III enthalten, mit den niedermolekularen und/oder oligomeren Verbindungen, welche die Allophanatgruppen IV enthalten, oder mit den oligomeren und/oder hochmolekularen erfindungsgemäßen Umsetzungsprodukten oder den oligomeren und hochmolekularen Verbindungen, welche die Allophanatgruppen II enthalten, vernetzen. In dieser Weise ist es möglich, völlig neue feststoffreiche Beschichtungsmittel, Klebstoffe und Dichtungsmassen oder Folien, insbesondere freitragende Lackfilme, herzustellen.

Besonders vorteilhafte erfindungsgemäße Bindemittel, Klebstoffe und Dichtungsmassen enthalten als Bindemittel die oligomeren und/oder hochmolekularen Verbindungen, welche die Allophanatgruppen II enthalten.

Darüber hinaus enthalten sie die üblichen und bekannten Aminoplastharze und/oder die vorstehend beschriebenen niedermolekularen und/oder oligomeren Verbindungen, bei denen nur ein Teil der Methylol- und/oder Methoximethylgruppen zu Allophanatgruppen III umgesetzt sind. Beispiele geeigneter üblicher und bekannter Aminoplastharze und erfindungsgemäßer Verbindungen sind die vorstehend beschriebenen.

Außer diesen Vernetzern können noch weitere Vernetzer vorhanden sein. Beispiele geeigneter weiterer Vernetzer sind Siloxangruppen enthaltende Verbindungen oder Harze, Anhydridgruppen enthaltende Verbindungen oder Harze, Epoxidgruppen enthaltende Verbindungen oder Harze, blockierte und/oder unblockierte Polyisocyanate und/oder Tris(alkoxycarbonylamino)triazine wie sie in den Patentschriften US-A-4 939 213,US-A-5 084 541, US-A-5 288 865 oder EP-A-0 604 922 beschrieben werden.

Je nach der Reaktivität des weiteren Vernetzers kann er den erfindungsgemäßen Beschichtungsmitteln, Klebstoffen und Dichtungsmassen direkt zugesetzt werden, wodurch ein sogenanntes Einkomponentensystem resultiert. Handelt es sich indes um einen besonders reaktiven Vernetzer, wie ein Polyisocyanat oder ein Epoxid, wird dieser im allgemeinen erst kurz vor der Verwendung den erfindungsgemäßen Beschichtungsmitteln, Klebstoffen und Dichtungsmassen zugesetzt. Hierbei resultiert ein sogenanntes Zwei- oder Mehrkomponentensystem.

Die erfindungsgemäßen Beschichtungsmittel, Klebstoffe und Dichtungsmassen können übliche und bekannte Zusatzstoffe in üblichen und bekannten, wirksamen Mengen enthalten.

Beispiele geeigneter Zusatzstoffe sind Polymere, Vemetzer, Katalysatoren für die Vernetzung, Initiatoren, insbesondere Photoinitiatoren, Pigmente, Farbstoffe, Füllstoffe, Verstärkerfüllstoffe, Rheologiehilfsmittel, Lösemittel, Netz- und Dispergiermittel, Entschäumer, Haftvermittler, Additive zur Verbesserung der Untergrundbenetzung, Additive zur Verbesserung der Oberflächenglätte, Mattierungsmittel, Verlaufmittel, Filmbildehilfsmittel, Trockenstoffe, Hautverhinderungsmittel, Lichtschutzmittel, Korrosionsinhibitoren, Biozide, Flammschutzmittel, Polymerisationsinhibitoren, insbesondere Photoinhibitoren, oder Weichmacher, wie sie beispielsweise auf dem Kunststoff- oder Lacksektor üblich und bekannt sind.

Die Auswahl der Zusatzstoffe richtet sich nach dem gewünschten Eigenschaftsprofil der erfindungsgemäßen Beschichtungsmittel, Klebstoffe und Dichtungsmassen sowie deren speziellen Verwendungszweck und kann daher vom Fachmann in einfacher Weise, gegebenenfalls unter Zuhilfenahme einfacher Vorversuche, getroffen werden.

Die erfindungsgemäßen Beschichtungsmittel können in wäßrigen, wäßrig organischen oder organischen Medien gelöst oder dispergiert oder als sogenannte "NAD" (non-aqueous dispersion) vorliegen. Außerdem können sie fein verteilt in fester Form als Pulverlacke oder in fester Formen in Wasser dispergiert als Pulverslurries vorliegen. Die jeweils erforderlichen Bestandteile der erfindungsgemäßen Beschichtungsmittel kann der Fachmann leicht anhand des vorgegebenen Eigenschaftsprofils (fest, flüssig, in organischen Lösemittel löslich, wasserlöslich usw.) auswählen.

Die erfindungsgemäßen Beschichtungsmittel lassen sich daher zahlreichen Verwendungszwecken zu führen. So können sie als Pulverlacke in der Industrielackierung, Autoserienlackierung oder in der Autoreparaturlackierung verwendet werden. Hierbei macht sich ihre ausgezeichnete Lagerstabilität vorteilhaft bemerkbar.

Die besonderen Vorteile der erfindungsgemäßen Beschichtungsmittel treten insbesondere in der Autoserienlackierung zutage. Hier können sie als Unterbodenschutz, Grundierungen, Antidröhnmassen, Füller, Steinschlagfüller, Basislacke, Unidecklacke und/oder Klarlacke verwendet werden. Mit ganz besonderem Vorteil werden sie als Klarlacke verwendet. Hierbei lassen sie sich problemlos nach dem Naß-in-naß-Verfahren applizieren. Nach dem Einbrennen zeigt sich ihre hohe Verträglichkeit mit allen üblichen und bekannten, insbesondere aber mit den erfindungsgemäßen, Basislacken. Die erfindungsgemäßen Klarlacke sind besonders witterungsstabil und kratzfest.

**Beispiele**

**1. Die Herstellung einer hochmolekularen erfindungsgemäßen Verbindung mit Allophanatgruppen II**

**1.1 Herstellung eines hydroxylgruppenhaltigen Polymethacrylats**

Die Herstellung des Polymethacrylats erfolgte in einem 4-Liter-Edelstahlreaktor mit Rührer, Rückflußkühler, einem Zulaufgefäß für Monomere und einem für den Initiator. Es wurden 930 g Solventnaphtha vorgelegt und auf 140 °C aufgeheizt. Innerhalb von 4,75 Stunden wurde der Initiatorzulauf, bestehend aus 167g Solventnaphtha und 167 g tert.-Butylperoxi-2-ethylhexanoat, gleichmäßig zudosiert. Innerhalb von vier Stunden wurde der Monomerenzulauf, bestehend aus 348 g Ethylhexylacrylat, 348 g Ethylhexylmethacrylat, 600 g Hydroxyethylmethacrylat und 369 g Styrol gleichmäßig zudosiert. Der Monomerenzulauf von 15 Minuten nach dem Initiatorzulauf gestartet. Während der Polymerisation hielt man die Temperaturen auf 140 °C. Nach zweistündiger Nachpolymerisation wurde die Reaktionsmischung abgekühlt und mit Solventnaphtha auf einen Festkörpergehalt von 60 Gew.-% eingestellt. Die Säurezahl betrug 4 und die Viskosität (original) 12 dPa.s..

**1.2 Die Transallophanatisierung des hydroxylgruppenhaltigen Polymethacrylats mit Allophansäureethylester**

469,03 Teile des hydroxylgruppenhaltigen Polyacrylats gemäß Beispiel 1.1 (60%-ig in Solventnaphtha), 102,4 Teile Allophansäureethylester und 36,7 Teile Cyclohexan wurden in einem geeigneten Stahlreaktor vorgelegt und auf 130 °C erhitzt. Bei Erreichen dieser Temperatur wurden 1,0 Teile Triisodecylphosphit und 7,4 Teile Solventnaphtha einzelnen zugegeben. Die resultierende Reaktionsmischung wurde während drei Stunden auf 130 °C erhitzt.

Danach wurden 0,5 Teile Dibutylzinndioxid und 7,4 Teile Solventnaphtha zugesetzt und die Reaktionsmischung weitere drei Stunden bei 130 °C gerührt. Nach dieser Zeit wurden erneut, 5 Teile Dibutylzinndioxid und 7,4 Teile Solventnaphtha hinzugegeben. Die resultierende Reaktionsmischung wurde weitere drei Stunden bei 130 °C gerührt.

Hiernach wurden flüchtige Reaktionsprodukte unter Vakuum bei 115 °C entfernt, und die resultierende Lösung des erfindungsgemäßen, die Allophanatgruppen II enthaltenden Polyacrylats wurde mit einer Mischung von Methoxipropylacetat und Pentylacetat auf einen Festkörpergehalt von 60 Gew.-% eingestellt. Die Hydroxylzahl (OHZ) des Produktes lag unter 5.

**1.3 Die Herstellung und die lacktechnische Prüfung eines erfindungsgemäßen Beschichtungsmittels**

64,8 Teile des erfindungsgemäßen Bindemittels gem. Beispiel 1.2, 9,5 Teile eines üblichen und bekannten Aminoplastharzes (Cymel 327; American Cyanamid), 1,6 Teile Tinuvin[R] 384 (UV-Absorber; Ciba Geigy), 0,8 Teile Tinuvin[R] 123 (Radikalfänger; Ciba Geigy), 1,6 Teile Nacure[R] 4575 (Katalysator, King Industries), 1,0 Teile eines handelsüblichen Verlaufsadditivs, gelöst in 0,2 Teilen Xylol, 3,4 Teile n- Butanol, 2,1 Teile Butylglykolacetat und 14,1 Teile Solventnaphtha wurde miteinander vermischt. Es resultierte ein Klarlack, welcher nach 16 Stunden bei 60 °C sowie nach 28 Tagen bei 40 °C keinen Anstieg der Viskosität zeigte. Dies belegte seine ausgezeichnete Lagerstabilität.

Der erfindungsgemäße Klarlack wurde mit einer Fließbecherpistole (Auslaufviskosität: 28s DIN 4 Becher) auf Prüftafeln, welche mit einem üblichen und bekannten Elektrotauchlack (20 mikrometer), einen üblichen und bekannten Füller (31 mikrometer) und einem üblichen und bekannten Basislack (17 mikrometer) beschichtet waren, appliziert. Hierbei wurde der Basislack vor der Applikation des Klarlacks aufgetragen und während zehn Minuten bei Raumtemperatur vor dem Auftrag des Klarlacks abgelüftet.

Nach seiner Applikation wurde der Klarlack ebenfalls während zehn Minuten bei Raumtemperatur abgelüftet. Danach wurden der Basislack und der Klarlack während zwanzig Minuten bei 140 °C eingebrannt. Die resultierende Klarlackschicht wies eine Schichtdicke von 28 mikrometer auf.

Lacktechnische Prüfungen:

An den mit dem erfindungsgemäßen Klarlack beschichteten Prüftafeln wurden die folgenden lacktechnischen Prüfungen durchgeführt.

**1. BART-Test (Chemikalienbeständigkeit)**

Der BART (BASF ACID RESISTANCE TEST) diente der Ermittlung der Beständigkeit von Lackoberflächen gegen Säuren, Laugen und Wassertropfen. Dabei wurde die Beschichtung auf einem Gradientenofen nach der Einbrennung weiteren Temperaturbelastungen ausgesetzt (30 min 40°C, 50°C, 60°C und 70°C). Zuvor wurden die Testsubstanzen (Schwefelsäure 1%ig, 10%ig, 36%ig; schweflige Säure 6%ig; Salzsäure 10%ig; Natronlauge 5%ig; VE (=vollentsalztes) Wasser - 1,2,3 bzw. 4 Tropfen) definiert mit einer Dosierpipette aufgebracht. Im Anschluß an die Einwirkung der Substanzen wurden diese unter fließendem Wasser entfernt, und die Beschädigungen wurden nach 24 h entsprechend einer vorgegebenen Skala visuell beurteilt:

| Benotung | Aussehen |
|---|---|
| 0 | kein Defekt |
| 1 | leichte Markierung |
| 2 | Markierung / Vermattung / keine Erweichung |
| 3 | Markierung / Vermattung / Farbtonveränderung / Erweichung |
| 4 | Risse / beginnende Durchätzung |
| 5 | Klarlack entfernt |

Es wurde jede einzelne Markierung (Spot) ausgewertet und das Ergebnis für jede Beschichtung in geeigneter Form (z.B. Notensummen für eine Temperatur) festgehalten.

Die Ergebnisse finden sich in der Tabelle 1.

Tabelle 1:

| Ergebnis der lacktechnischen Prüfung nach dem BART-Test Erfindungsgemäßer Klarlack | | | | | |
|---|---|---|---|---|---|
| Temperatur (°C) | **40** | **50** | **60** | | |
| H$_2$SO$_4$ 1%ig | 0 | 0 | 0 | | |
| H$_2$SO$_4$ 10%ig | 0 | 0 | 0 | | |
| H$_2$SO$_4$ 36%ig | 0 | 0 | 0 | | |
| HCl 10%ig | 0 | 0 | 0 | | |
| H$_2$SO$_3$ 5%ig | 0 | 0 | 0 | | |
| NaOH 5%ig | 0 | 0 | 0 | | |
| VE-Wasser 1 | 0 | 0 | 1 | | |
| VE-Wasser 2 | 0 | 0 | 0 | | |
| VE-Wasser 3 | 0 | 0 | 0 | | |
| VE-Wasser 4 | 0 | 0 | 0 | | |
| **Summe Säure** | **0** | **0** | **0** | | |
| **Summe Wasser** | **0** | **0** | **1** | | |

## 2. Sandtest und Bürstentest (Kratzbeständigkeit)

### 2.1 Sandtest

Mit diesem Prüfverfahren wird die Beständigkeit (scratch resistance) von Lackoberflächen (Klarlacke und Decklacke) gegen Waschbürstenkratzer geprüft.

Das Verfahren ist eine gute Nachstellung der Belastung einer Lackoberfläche in einer Waschanlage

Beim Sandtest wurde die Lackoberfläche mit Sand belastet (20g Quarz-Silbersand 1.5-2.0 mm). Der Sand wurde in einen Polyethylen-Becher (Boden plan abgeschnitten) gegeben, der fest auf der Prüftafel befestigt wurde. Es wurden die gleichen Prüftafeln, wie oben im Bürstentest beschrieben, verwendet. Mittels eines Motorantriebes wurde die Tafel mit dem Becher und dem Sand in Schüttelbewegungen versetzt. Die Bewegungen des losen Sandes verursachte dabei die Beschädigung der Lackoberfläche (100 Doppelhübe in 22s). Nach der Sandbelastung wurde die Prüffläche vom Abrieb gereinigt, unter einem kalten Wasserstrahl vorsichtig abgewischt und anschließend mit Druckluft getrocknet. Gemessen wurde der Glanz nach DIN 67530 vor und nach Beschädigung bei 20°.

Die Ergebnisse finden sich in der Tabelle 2.

### 2.2 Bürstentest

Die Kratzfestigkeit der ausgehärteten Beschichtungen wurde mit Hilfe des in Fig. 2 auf Seite 28 des Artikels von P. Betz und A. Bartelt, Progress in Organic Coatings, 22 (1993), Seiten 27 - 37, beschriebenen BASF-Bürstentests, der allerdings bezüglich des verwendeten Gewichts (2000 g statt der dort genannten 280 g) abgewandelt wurde, folgendermaßen beurteilt:

Bei dem Test wurde die Lackoberfläche mit einem Siebgewebe, welches mit einer Masse belastet wurde, geschädigt. Das Siebgewebe und die Lackoberfläche wurden mit einer Waschmittel-Lösung reichlich benetzt. Die Prüftafel wurde mittels eines Motorantriebs in Hubbewegungen unter dem Siebgewebe vor- und zurückgeschoben.

Zur Herstellung der Prüftafeln wurden zunächst eine Elektrotauchlackierung mit einer Schichtdicke von 18 - 22 µm, dann ein Füller mit einer Schichtdicke von 35 - 40 µm, dann ein schwarzer Basislack mit einer Schichtdicke von 20 - 25 µm und abschließend der erfindungsgemäße Klarlack mit einer Schichtdicke von 40 - 45 µm appliziert und jeweils gehärtet. Die Tafeln wurden nach Applikation der Lacke mindestens 2 Wochen bei Raumtemperatur gelagert, bevor die Prüfung durchgeführt wurde.

Der Prüfkörper war mit Nylon-Siebgewebe (Nr. 11, 31 µm Maschenweite, Tg 50 °C) bespanntes Radiergummi (4,5 x 2,0 cm, breite Seite senkrecht zur Kratzrichtung). Das Auflagegewicht betrugt 2000 g.

Vor jeder Prüfung wurde das Siebgewebe erneuert, dabei war die Laufrichtung der Gewebemaschen parallel zur Kratzrichtung. Mit einer Pipette wurde ca. 1 ml einer frisch aufgerührten 0,25%igen Persil-Lösung vor dem Radiergummi aufgebracht. Die Umdrehungszahl des Motors wurde so eingestellt, daß in einer Zeit von 80 s 80 Doppelhübe ausgeführt wurden. Nach der Prüfung wurde die verbleibende Waschflüssigkeit mit kaltem Leitungswasser abgespült und die Prüftafel mit Druckluft trockengeblasen. Gemessen wurde der Glanz nach DIN 67530 vor und nach Beschädigung (Meßrichtung senkrecht zur Kratzrichtung).

Die Ergebnisse des Tests finden sich ebenfalls in der Tabelle 2.

Tabelle 2:

| Ergebnisse des Sandtests und des Bürstentests | | |
|---|---|---|
| **Glanzwerte[a]** | **Erfindungsgemäßer Klarlack Sandtest** | **Erfindungsgemäßer Klarlack Bürstentest** |
| Ausgangsglanz | 92 | 90 |
| Restglanz | 60 | 58 |
| Glanz nach 2h, 40 °C | 64 | 61 |
| Glanz nach 2h, 60 °C | 65 | 64 |

a) gemessen bei 20°C

Die Ergebnisse der Tabelle 2 belegen die hohe Kratzfestigkeit des erfindungsgemäßen Klarlacks und sein gutes Reflow-Verhalten.

**3. Gitterschnittprüfung**

Der Gitterschnittprüfung wurde gemäß DIN ISO 2409: 1994-10 durchgeführt. Es wurde keine Enthaftung beobachtet (GT-01), was die vorzügliche Haftung des erfindungsgemäßen Klarlacks auf dem Basislack belegt.

**Patentansprüche**

**1.** Oligomere und hochmolekulare Umsetzungsprodukte von Allophansäurealkyl- und -arylestern mit nucleophilen Verbindungen, wobei der Begriff "oligomer" bedeutet, daß die betreffende Verbindung im Mittel aus 3 bis 10 sich wiederholenden identischen Grundbausteinen aufgebaut ist, und der Begriff "hochmolekular" bedeutet, daß die betreffende Verbindung im Mittel aus mehr als 10 sich wiederholenden identischen Grundbausteinen aufgebaut ist.

**2.** Verwendung der oligomeren und/oder hochmolekularen Umsetzungsprodukte von Allophansäurealkyl- und -arylestern mit nucleophilen Verbindungen gemäß Anspruch 1 als Bindemittel für Beschichtungsmittel, Klebstoffe und Dichtungsmassen oder für die Herstellung von Folien.

**3.** Verwendung der oligomeren Umsetzungsprodukte von Allophansäurealkylund arylestern mit nucleophilen Verbindungen gemäß Anspruch 1 als Vernetzer für Beschichtungsmittel, Klebstoffe und Dichtungsmassen oder für die Herstellung von Folien.

**4.** Beschichtungsmittel, Folien, Klebstoffe und Dichtungsmassen, welche die oligomeren und/oder hochmolekularen Umsetzungsprodukte von Allophansäureestern mit nucleophilen Verbindungen gemäß Anspruch 1 enthalten.

**5.** Verfahren zur Herstellung der oligomeren und hochmolekularen Umsetzungsprodukte gemäß Anspruch 1 durch Transallophanatisierung von hydroxylgruppenhaltigen Oligomeren und/oder Polymeren (Nucleophilen) mit Allophansäurealkyl- und -arylestern.

**6.** Verfahren zur Herstellung von niedermolekularen und oligomeren Verbindungen mit Allophanatseiten- und/oder -endgruppen durch Umsetzung von Aminoplastharzen mit Allophansäurealkyl- und -arylestern, wobei der Begriff "oligomer" bedeutet, daß die betreffende Verbindung im Mittel aus 3 bis 10 sich wiederholenden identischen Grundbausteinen aufgebaut ist.

7. Verfahren zur Herstellung von niedermolekularen und oligomeren Verbindungen mit Allophanatseiten- und/oder -endgruppen durch Umsetzung von Polyisocyanaten mit Hydroxyalkyl- und arylallophanaten, wobei der Begriff "oligomer" bedeutet, daß die betreffende Verbindung im Mittel aus 3 bis 10 sich wiederholenden identischen Grundbausteinen aufgebaut ist.

**Claims**

1. Oligomeric and high molecular mass products of reaction of alkyl and aryl allophanates with nucleophilic compounds, the term "oligomeric" meaning that the compound in question is composed on average of from 3 to 10 repeating identical basic building blocks, and the term "high molecular mass" meaning that the compound in question is composed on average of more than 10 repeating identical basic building blocks.

2. Use of the oligomeric and/or high molecular mass products of reaction of alkyl and aryl allophanates with nucleophilic compounds, in accordance with Claim 1, as binders for coating compositions, adhesives and sealing compounds or for producing films.

3. Use of the oligomeric products of reaction of alkyl and aryl allophanates with nucleophilic compounds, in accordance with Claim 1, as cross-linkers for coating compositions, adhesives and sealing compounds or for producing films.

4. Coating compositions, films, adhesives and sealing compounds which comprise the oligomeric and/or high molecular mass products of reaction of allophanic esters with nucleophilic compounds, in accordance with Claim 1.

5. Process for preparing the oligomeric and high molecular mass products of reaction, in accordance with Claim 1, by transallophanatization of hydroxyl-containing oligomers and/or polymers (nucleophiles) with alkyl and aryl allophanates.

6. Process for preparing low molecular mass and oligomeric compounds containing lateral and/or terminal allophanate groups by reacting amino resins with alkyl and aryl allophanates, the term "oligomeric" meaning that the compound in question is composed on average of from 3 to 10 repeating identical basic building blocks.

7. Process for preparing low molecular mass and oligomeric compounds containing lateral and/or terminal allophanate groups by reacting polyisocyanates with hydroxyalkyl and aryl allophanates, the term "oligomeric" meaning that the compound in question is composed on average of from 3 to 10 repeating identical basic building blocks.

**Revendications**

1. Produits oligomères et à haut poids moléculaire de conversion d'alkylesters de l'acide allophanique et d'arylesters de l'acide allophanique qui présentent des composés nucléophiles, le terme "oligomère" signifiant que le composé concerné est composé en moyenne de 3 à 10 briques de base qui se répètent à l'identique et le terme "à haut poids moléculaire" signifiant que le composé concerné est composé en moyenne de plus de 10 briques de base qui se répètent à l'identique.

2. Utilisation des produits oligomères et/ou à haut poids moléculaire de conversion d'alkylesters de l'acide allophanique et d'arylesters de l'acide allophanique, qui présentent des composés nucléophiles selon la revendication 1 comme liant pour des agents de revêtement, des adhésifs et des pâtes d'étanchéité ou pour la fabrication de films.

3. Utilisation des produits oligomères de conversion d'alkylesters de l'acide allophanique et d'arylesters de l'acide allophanique qui présentent des composés nucléophiles selon la revendication 1 comme agents de réticulation pour des agents de revêtement, des adhésifs et des pâtes d'étanchéité ou pour la fabrication de films.

4. Agents de revêtement, films, adhésifs et pâtes d'étanchéité, qui contiennent les produits oligomères et/ou les produits à haut poids moléculaire de conversion d'esters de l'acide allophanique qui présentent des composés nucléophiles selon la revendication 1.

5. Procédé pour préparer les produits de conversion oligomères et les produits à haut poids moléculaire selon la revendication 1 par transallophanation d'oligomères et/ou de polymères (nucléophiles) qui contiennent des groupes hydroxyle avec des alkylesters de l'acide allophanique et des arylesters de l'acide allophanique.

6. Procédé pour préparer des composés à bas poids moléculaire et des composés oligomères qui présentent des groupes latéraux et/ou terminaux allophanate par conversion de résines aminoplastiques avec des alkylesters de l'acide allophanique et des arylesters de l'acide allophanique, le terme "oligomère" signifiant que le composé concerné comprend en moyenne entre 3 et 10 briques de base qui se répètent à l'identique.

7. Procédé pour préparer des composés à bas poids moléculaire et des composés oligomères qui présentent des groupes latéraux et/ou terminaux allophanate par conversion de polyisocyanates avec des allophanates d'hydroxyalkyle et des allophanates d'aryle, le terme "oligomère" signifiant que le composé concerné comprend en moyenne entre 3 et 10 briques de base qui se répètent à l'identique.